# EUROPEAN PATENT APPLICATION

(11) **EP 3 015 463 A1**
(43) Date of publication of application: **04.05.2016**
(21) Application number: 14191075.2
(22) Date of filing: 30.10.2014
(51) Int. Cl.: C07D 307/48

(54) **Crystallization of furanic compounds**

(71) Applicant: Nederlandse Organisatie voor toegepast- natuurwetenschappelijk onderzoek TNO, 2595 DA 's-Gravenhage (NL)
(72) Inventor: URBANUS, Jan, Harm, 2595 DA 's-Gravenhage (NL); VERDOES, Dirk, 2595 DA 's-Gravenhage (NL); ROA ENGEL, Carol Andrea, 2595 DA 's-Gravenhage (NL)
(74) Representative: V.O.

(57) **Abstract**

The invention relates to a method for preparing furanic compound crystals from sugars comprising the steps of
- converting sugar or sugar alcohol into a furanic compound in a reaction mixture comprising an organic solvent, thereby obtaining a product mixture comprising a furanic compound dissolved in said organic solvent and water;
- preferably removing water from the product mixture; and
- simultaneously crystallizing the furanic compound and the organic solvent by cooling the product mixture, thereby obtaining a slurry comprising solvent crystals and furanic compound crystals; and
- separating the solvent crystals and the furanic compound crystals.

## Description

The invention is directed to a method for preparing furanic compounds and a mixture of furanic compound crystals and organic solvent crystals obtained therein.

Furanic compounds, such as furfural (FF) and hydroxymethylfurfural (HMF), are nowadays becoming more and more important as renewable building blocks for chemical industry. Furanic compounds are typically produced from sugars. For example, HMF can be prepared by the conversion of fructose into HMF. Furfural has been produced from sugars on a commercial scale for several decades, while efforts are recently made to also scale-up HMF production.

The current commercial process with which furanic compounds are produced is by sugar conversion in an aqueous media, which resulted in a yield of 50-60%. However, it has recently been found that furanic compounds can also be produced in an organic solution. An advantage of producing furanic compounds from sugars in an organic solvent instead of in water is that less by-products is formed and that a higher yield can be obtained, typically over 90%. The organic solvent used in converting sugar into furanic compounds must be aprotic. This facilitates solubilization of the sugar feedstock and further to enable the conversion into furans (in particular when the conversion is acid-catalyzed).

Although conversion in an organic solution has been studied at small scale, furanic compounds have not yet been successfully produced at commercial scale. To achieve this, a number of problems still need to be solved.

One problem of sugar conversion to furanic compounds in an organic solvent is the separation of the furanic compound from the organic solvent. The organic solvents are known to interact with furanic compounds such that separation is difficult.

In the past years, several techniques have been tested to solve this problem.

For example, FR 2 669 635 describes a process for preparing HMF in an aprotic solvent, after which water and a second organic solvent are added to extract the HMF formed. Thus, two liquid phases are formed, which are separated. HMF is crystallized by cooling the second liquid phase. WO 2013/024162 describes a procedure for purifying HMF by crystallization at low temperature from an organic solvent having a freezing point of -50°C or lower by cooling the solution to a temperature of between 0 and -40°C. Precipitated solid HMF is isolated by filtering the obtained suspension. Given the nature of most solvents with the specified melting point, this procedure typically applies to the process described by FR 2 669 635, where a solvent is added to the aprotic solvent in which the reaction takes place. Although these and other separation techniques of the prior art, such as distillation, membrane assisted pervaporation, extraction into other solvents, adsorption and chromatography, have been proven to effectively isolate the HMF, these techniques have resulted in other problems. The processes are energy consuming, produce additional waste (e.g. due to additional organic solvent use) and/or require expensive equipment. Further, they can often not be easily implemented at full industrial scale at acceptable energy and investment costs.

Accordingly, there is still a need for alternative methods for preparing and processing HMF. In particular, there is a need for a process wherein HMF and the organic solvent in which it is prepared can be efficiently separated, while the process can at the same time be implemented at full industrial scale at acceptable energy and investment costs.

It is therefore an object of the invention to provide a process for preparing furanic compounds from sugar in an organic solvent, wherein the furanic compound can be efficiently separated from the reaction mixture. More in particular, an object of the invention is to achieve this by a relatively simple and energy efficient process that can easily be scaled up to industrial scale.

This object was met by providing a method for preparing furanic compound crystals from sugars comprising the steps of
- converting sugar or sugar alcohol into a furanic compound in a reaction mixture comprising an organic solvent, thereby obtaining a product mixture comprising a furanic compound dissolved in said organic solvent and water;
- optionally further converting the furanic compound; and
- preferably removing water from the product mixture; and
- simultaneously crystallizing the (converted or unconverted) furanic compound and the organic solvent by cooling the product mixture, thereby obtaining a slurry comprising solvent crystals and furanic compound crystals; and
- separating the solvent crystals and the furanic compound crystals.

The inventors found that simultaneous crystallization of the product and the solvent resulted in a very efficient separation To the inventors' knowledge, simultaneous crystallization by cooling (also known as eutectic freeze crystallization) has never been considered before as a viable separation technique in the field of furanic compound synthesis. Up to now, eutectic freeze crystallization has only been used for removal of salt and/or organic contaminants from aqueous streams, for example in waste water treatment. The inventors found that this technique could also be applied for isolation and separation of furanic compounds from an organic solvent after sugar conversion. The efficient separation of the furanic compound and the organic solvent is also illustrated in the Examples.

The inventors further realized that it is not desirable to directly subject the product mixture obtained in the conversion step to simultaneous crystallization. In such a case, complications would arise during crystallization and/or separation of the product and solvent. For example, a mixture of three solids may be formed, which considerably complicates the separation step. The inventors realized that such complications were caused by the presence of water in the product mixture. Water is formed as a byproduct during the conversion of sugar into furanic compounds and is therefore necessarily present in the product mixture obtained in the conversion step. By removing water from the product mixture prior to simultaneous crystallization, the afore-mentioned complications can thus be avoided. Thus, a particular efficient crystallization and/or separation is achieved.

Further, it was found that by selecting a suitable combination of a furanic compound and an organic solvent, the temperature at which crystallization occurs can be controlled. As such, a crystallization temperature may be used in the process (*e.g*. just below 20 °C), such that the energy consumption for cooling will be small. Thus, the process of the invention provides for an energy efficient separation process. Further, since the method only requires relatively simple crystallization and cooling equipment, the method can be suitably scaled up to industrial scale without the need of expensive equipment. The process can suitably be conducted in a continuous process, although it may also be conducted semi-continuous or batch wise.

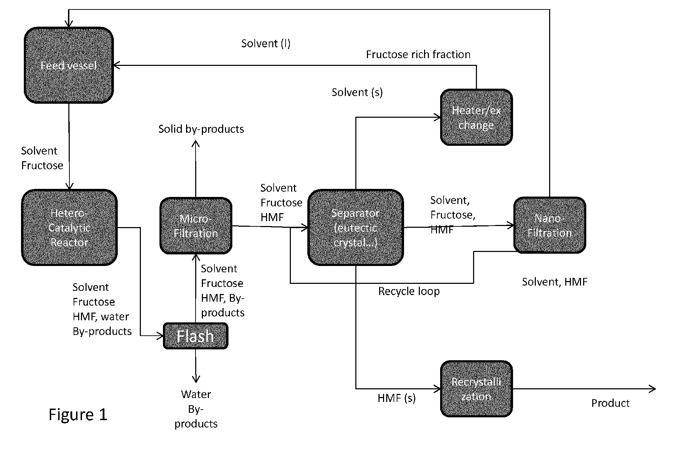

Figure 1 shows a schematic representation of an embodiment according to the method of the invention. In this embodiment, fructose is converted to HMF. Water is removed by evaporation (flash) from the product mixture obtained in the conversion step, after which the mixture is subjected to microfiltration and simultaneous crystallization.

The term "furanic compound" refers to a compound having one or more furan groups. A furan group is a five-membered aromatic ring with four carbon atoms and one oxygen atom.

The term "product mixture" as used herein either refers to the product mixture obtained by converting sugar into the furanic compound (in case the furanic compound is not subjected to a further conversion) or to the product mixture obtained by further converting the furanic compound (in case such a further (second) conversion step is conducted.

The term "trialkylphosphine oxide" refers to a group of compounds with the formula O=P(R)₃, wherein R is an alkyl group. The alkyl group is preferably a C₁₋₂₀ alkyl, preferably a C₄₋₁₂ alkyl, more preferably a C₆₋₁₀ alkyl. Particular good results were obtained using tri-n-octylphosphine oxide (TOPO) as the organic solvent.

The term "dialkylsulfoxide" refers to a group of compounds with the formula O=S(R)₂, wherein R is an alkyl group. The alkyl group is preferably a C₁₋₈ alkyl, preferably a C₁₋₆ alkyl, for example dimethylsulfoxide (DMSO) or dibutylsulfoxide (DBSO).

The term "eutectic point" as used herein refers to the specific temperature (referred to as the "eutectic temperature") and the specific ratio between the organic solvent and furanic compound (referred to as the "eutectic composition" or "eutectic ratio") in the product mixture at which simultaneous crystallization of the organic solvent and the furanic compound occurs. Although the product mixture may have more than one eutectic point (*e.g*. due to the presence of other components in the mixture, such as byproducts or reactants of the first and/or second conversion reaction), the eutectic point as referred to in the present invention only concerns the eutectic point at which the organic solvent and the furanic compound simultaneously crystallize.

The different steps of the method of the invention will be discussed in more detail below.

In the first step, sugar is converted into a furanic compound in a reaction mixture comprising an organic solvent. This step may also be referred to as the sugar conversion or first conversion step. The reaction mixture may further comprise a catalyst to promote the conversion of the sugar into the furanic compound and/or to promote the further conversion of the furanic compound if such a step is conducted. The catalyst may be in solid form (in case of heterogeneous catalysis) or in dissolved form (in case of homogeneous catalysis). The homogeneous catalyst is preferably volatile and may have a boiling point of below 100°C. An advantage of such a volatile catalyst is that they can be easily removed from the reaction mixture, for example by flashing. The homogeneous catalyst may be an acidic catalyst.

The conversion reaction can be conducted at a temperature of at least 75 °C, but is typically conducted at a temperature of at least 100 °C. The pressure during the conversion reaction may be in the range of 1-100000 kPa, preferably 1-1000 kPa. The reaction mixture typically comprises at least 0.1 wt.% fructose, preferably 5-15 wt.% sugar.

Any sugar or sugar alcohol that can be suitably converted into furanic compounds may be used in the reaction mixture. For example, the sugar may be a sugar with 4, 5 or 6 carbon atoms, *i.e*. a C4, C5 or C6 sugar. An example of a suitable sugar alcohol is erythritol (which can *e.g.* be converted into furan). Specific examples of suitable sugars are fructose, glucose, xylose, arabinose and galactose. Fructose is commonly used for HMF production. Modified versions of sugars may also be used. Such forms can be obtained by reacting the sugar (*e.g.* fructose) with a suitable alcohol and/or acid. The sugars may originate from any source, *e.g.* from cellulose, hemicellulose, starch, sucrose, inuline, pectin or sugar alcohols.

The furanic compound may be selected from the group consisting of hydroxymethylfurfural (HMF); furfural (FF); furandicarboxylic acid (FDCA); alkoxymethylfurfural, such as methoxymethylfurfural (MMF); and haloalkylfurfurals, such as 5-chloromethylfurfural. Preferably, the furanic compound is HMF.

The reaction mixture may be a one-phase system or a two-phase system.

In case of a one-phase system, the reaction mixture comprises a single liquid phase, which mainly (>50wt.%) consist of the organic solvent. In such a system, the sugar should be dissolved in the organic solvent. Therefore, the organic solvent should be sufficiently aprotic. Examples of suitable aprotic solvents are trialkylphosphine oxides (such as TOPO), dialkylsulfoxides (such as DMSO and DBSO) and sulfolane. Preferably, dimethyl sulfoxide is used as an aprotic organic solvent. The conversion reaction in a one-phase system will convert sugar into the furanic compound, which will also dissolve in the organic solvent. Furthermore, water is formed as a by-product in the conversion of sugar to furanic compounds. Accordingly, a product mixture is obtained comprising the furanic compound, which will be dissolved in the organic solvent. The product mixture will further comprise water (which may dissolve or be miscible with the organic solvent) and possibly unreacted sugar (dissolved in the organic solvent).

In case of a two-phase system, the reaction mixture comprises two immiscible liquid phases. The reaction mixture may for example be an emulsion. The first liquid phase is an aqueous phase in which the sugar is dissolved. The second liquid phase is an organic phase, which mainly (>50wt.%) consists of the organic solvent. The organic solvent should be immiscible with water to be able to form the two-phase system. Examples of such organic solvents are certain dialkyl sulfoxides with a C3 or higher alkyl group (such as DBSO), trialkyl phosphines (such as TOPO) and sulfolane. Since sugar is present in the aqueous phase, the conversion reaction in a two-phase system takes place in the aqueous phase. Thus, the furanic compound is also formed in this phase. However, once formed, the furanic compound will be extracted to the organic phase. The conversion reaction thus results in a two-phase product mixture. The two phases are then separated to obtain the (organic) product mixture and an aqueous waste stream (comprising most unreacted sugar). The product mixture obtained after separating the two liquid phases comprises the furanic compound, which will be dissolved in the organic solvent. The product mixture may further comprise water (as a result of the aqueous phase with which it was in contact in the two-phase system).

The furanic compound obtained in sugar conversion may subsequently be further converted (prior to crystallization). This step may also be referred to as the second conversion step. The compound thus obtained may be referred to herein as the converted furanic compound. The conversion reaction in this step is typically conducted in the same solvent as in which the conversion from sugar (or sugar alcohol) took place, preferably in the mixture obtained in the sugar conversion step. The second conversion step is preferably conducted at a temperature of at least 25 °C and may be conducted at a temperature lower than at which sugar conversion takes place.

The furanic compound may be converted further by modifying one or more side groups of the furan ring in the furanic compound. For example, one or more side groups of the furan ring may be subjected to oxidation (e.g. modifying an alcohol group to an aldehyde), reduction (such as hydrogenation or hydrogenolysis), removal (such as decarbonylation or decarboxylation), addition (such as carboxylation; esterification or etherification, *e.g.* with a C1-C8 alcohol), substitution (such as halogenation or amination). The compound obtained by the modification reaction is still a furanic compound. For example, the converted compound may be one of furan carboxylic acids, esters of furan carboxylic acids, ethers of furan carboxylic acids, furan hydroxymethyls, furan alkoxymethyls, furan aldehydes, furan methylamines, furan carboxamides and haloalkylfurans. Specific examples of furanic compounds obtained by sugar conversion and a subsequent modification reaction are furoic acid; 2,5-furandicarboxylic acid; furfuryl alcohol; 2,5-bis(hydroxymethyl)furan; 2,5-diformylfuran; 2,5-bis(aminomethyl)furan; 5-hydroxymethyl-furan-2-carboxylic acid amide; 5-formyl-2-furancarboxylic acid; chloromethyl furfural; methylfuroate; and dimethyl furandicarboxylate. An example of modifying one of the side groups of a furanic compound by oxidation is the conversion of HMF into furan dialdehyde. Such an oxidation is normally conducted in water or acetic acid. However, in a preferred embodiment, the oxidation is conducted in DMSO, such that the conversion steps and simultaneous crystallization can all be conducted in the same solvent.

The furanic compound may also be converted by subjecting the furanic compound to a cycloaddition reaction. In this case, the converted furanic compound may be an aromatic compound. For example, the converted compound may be a mono-, di- or tri-substituted benzoic acid. Specific examples of furanic compounds obtained by sugar conversion and a subsequent cycloaddition are benzoic acid, phthalic acids (para, meta or ortho), mellitic acid, hemimellitic acid, hydroxybenzoic acids (para, meta or ortho) and cyanobenzoic acids (para, meta or ortho).

Although the compound obtained in the second conversion step may not necessarily be a furanic compound (especially in case of cycloaddition), the term "furanic compound" is used herein below both to refer to the (unconverted) furanic compound (in case no second conversion is conducted) and to the converted furanic compound (in case a second conversion is conducted), unless specifically noted otherwise.

As also noted above, the mixture obtained by sugar conversion (and optionally subsequent second conversion) is referred to as the product mixture.

Prior to simultaneous crystallization, water is preferably removed from the product mixture. This may for example be conducted by evaporation of water, preferably by flash evaporation. Since the product mixture typically has a relatively high temperature due to the heating that is typically applied during the conversion reaction(s), such evaporation is very efficient when conducted prior to crystallization. As described above, removal of water is important for an efficient crystallization and separation further on in the process. In particular, it may provide for one less phase to separate during crystallization. Preferably, the product mixture comprises less than 1 wt%, more preferably less than 0.1 wt.% water after water removal.

Besides water removal, the product mixture may be subjected to one or more other processing steps before simultaneously crystallizing the furanic compound and organic solvent. In particular, such steps may be conducted to remove certain material from the product mixture, such as solids, waste material (*e.g.* humins, by-products formed in the conversion of sugar to furanic compounds), catalyst and/or sugar. Such removal is advantageous, because these materials may interfere in the simultaneous crystallization and/or separation steps. In addition, feeding back certain materials before simultaneous crystallization may be advantageous for their reuse in the conversion step, *e.g.* in case of catalysts or unreacted sugar.

Specific examples of steps conducted before simultaneous crystallization are evaporation (*e.g.* to remove catalysts or solvent) and steps wherein material other than product or solvent is removed from the product mixture, such as filtration, for example microfiltration (*e.g.* for removing solid waste and/or solid catalyst) or nanofiltration (*e.g.* for removing sugar).

Thus, the product mixture may be subjected to one or more removal steps, for example filtration steps, wherein solid material is removed from the product mixture prior to simultaneous crystallization. For example, microfiltration may be conducted to remove solid material, such as solid waste or solid catalyst. Further, nanofiltration may be used to remove sugar.

Evaporation may be conducted before simultaneous crystallization not only to remove water, but also to remove volatile compounds that may be present in the product mixture such as volatile catalysts. The catalyst may be collected and reused after evaporation. Further, evaporation may be conducted to remove organic solvent or furanic compound in case it would be desirable for simultaneous crystallization if the product mixture has a different ratio of furanic compound to organic solvent (as also explained below).

Sugar may be recovered from the product mixture prior to simultaneous crystallization. The sugar thus obtained can be reused in the conversion step. Sugar may be removed in any suitable way known in the art, including nanofiltration. Sugar may also be removed from the mother liquor obtained after simultaneous crystallization, as described further below.

Further, a 'regular' crystallization step may be conducted prior to simultaneous crystallization, as described further below.

After having conducted water removal and possibly one or more of the further processing steps described above, the product mixture is subjected to simultaneous crystallization. During simultaneous crystallization, the mixture may also be referred to as the crystallization mixture. Simultaneous crystallization is conducted by cooling the product mixture such that the furanic compound and the organic solvent simultaneously crystallize from the product mixture. This step thus results in a slurry (*i.e*. a mixture of liquid and crystals) comprising solvent crystals and furanic compound crystals. The slurry further comprise a liquid, which liquid comprises at least one, but typically both of liquid furanic compounds and liquid organic solvent. This liquid is herein also referred to as the mother liquor. It will be understood that both the furanic compound and the organic solvent are valuable compounds that are typically reused.

Simultaneous crystallization is achieved by cooling, *i.e*. by lowering the temperature of the product mixture. Simultaneous crystallization will occur when the temperature of the product mixture is cooled to a sufficiently low temperature, *i.e*. cooled to the eutectic temperature of the product mixture or below. Simultaneous crystallization will only occur at a certain ratio of the furanic compound and organic solvent, *viz*. the eutectic composition. When cooling the mixture to (or below) its eutectic temperature at a composition other than the eutectic composition, either the furanic compound or the organic solvent will crystallize. This will effectively remove this solid component from the liquid mixture. As a result, the ratio of the organic compound and organic solvent will change and come closer to the ratio of the eutectic composition. The step where the composition of the mixture is shifted to the eutectic composition can be done in a separate step preceding the simultaneous crystallization (*e.g.* in a separate crystallizer) or be conducted in the same crystallizer as in which the simultaneous crystallization takes place. Eventually, the ratio will be equal to that of the eutectic composition. At this point, simultaneous crystallization will occur.

Simultaneous crystallization may be achieved by cooling the product mixture to a temperature equal or up to 25 °C, preferably equal or up to 10 °C below the eutectic point of the product mixture, more preferably 0.005 to 5 °C, even more preferably 0.05 to 3 °C below the eutectic point. It was found that cooling the product mixture only a few degrees below the eutectic point is desirable to control crystallization. Cooling to a temperature too far under the eutectic point may result in a sudden and abrupt crystallization, which may damage the crystallizer. Furthermore, cooling to temperature well below the eutectic point is typically not desirable, because energy would in such a case be needlessly wasted on cooling.

The organic solvent and the furanic compound may be simultaneously crystallized by cooling the product mixture to a temperature of -50 to 100°C, preferably -30 to 50°C, even more preferably -20°C to 30 °C, most preferably 0 to 20 °C. Cooling to such relatively mild temperatures is desirable, because the energy needed for cooling will be relatively low, such that an energy efficient process is obtained. Furthermore, such temperatures are desirable, because they can be achieved by standard methods and equipment for crystallization and cooling.

For the above cooling temperatures to be viable, the eutectic temperature of the product mixture should be in the same temperature ranges as given above for cooling. This can amongst others be achieved by selecting a suitable combination of the furanic compound and organic solvent to be used.

The eutectic temperature is for an important part determined by the melting point of the furanic compound and the organic solvent. Typically, the eutectic temperature will lie below the lowest melting point of the two compounds. Accordingly, the choice for the organic solvent will depend on the furanic compound that is to be prepared, and in particular its melting point. For example, HMF has a melting point of 30-34 °C, while furfural has a melting point of -37 °C (it may be desirable to convert furfural to furoic acid prior to simultaneous crystallization). In case furfural is the furanic compound, the organic solvent should therefore have a relatively high melting point, preferably of at least 30 °C to avoid a eutectic point having an undesirably low eutectic temperature. This will be less the case for HMF, for which it was found that DMSO (with a melting point of 19°C) can be very suitably used. Further, furoic acid and furandicarboxylic acid have a melting point above 150 °C, such that solvent with a relatively low melting point can be suitably used. Thus, the choice for a suitable organic solvent much depends on the melting point of the furanic compound to be isolated.

Although it will be evident from the above that the organic solvent's melting point will depend on the furanic compound prepared, the organic solvent will generally have a melting point of at least -30°C, typically at least 0°C, for example at least 15°C. Such values are generally preferred to avoid the eutectic temperature to become too low. Further, the organic solvent will generally have a melting point below 100°C, typically below 80°C, for example below 50°C. Such maximum values are generally preferred to avoid operational problems whilst dissolving sugars in the solvent prior to the conversion reactions. Examples of solvents that may be used in the method of the invention are trialkylphosphine oxides (such as TOPO), dialkylsulfoxides (such as DMSO and DBSO) and sulfolane.

As described above, the choice for the organic solvent may further depend on the type of reaction mixture (one-phase or two-phase), which determines whether an aprotic organic solvent or an organic solvent that is immiscible with water should be used.

Particularly desirable combinations of organic solvents and furanic compounds were found to be HMF or FF as the furanic compound in combination with trialkylphosphine oxides (in particular TOPO), dialkylsulfoxides (in particular DMSO and DBSO) and sulfolane. These combinations result in a desirable eutectic temperature, as also illustrated in the Examples.

Besides the eutectic temperature, the eutectic composition is of relevance for efficient simultaneous crystallization. In view of energy efficiency and efficient separation of the resulting crystals, it is generally undesirable to feed a product mixture to the crystallizer in which simultaneous crystallization takes place if the product mixture has an organic solvent to furanic compound ratio that is far off from the eutectic point of the product mixture. In such a case, one would first have to singly crystallize much solvent before reaching the eutectic point.

First, a suitable combination of furanic compound and organic solvent may be chosen to achieve a desirable ratio. Further, if possible, a desirable ratio may also be achieved by carrying out the conversion reaction of sugars to furanic compounds at such ratios that no adjustments are required. Otherwise, measures may be taken in order to shift the ratio between the organic solvent and the furanic compound (organic/furanic ratio), in order to arrive sooner at the eutectic composition of the product mixture. This may be either achieved by removing organic solvent (in case the solvent/furanic ratio is too high compared to the eutectic point) or by removing furanic acid (in case the solvent/furanic ratio is too low). Such removal can be achieved by conducting a concentration step or a crystallization step, as described below prior to simultaneous crystallization.

A concentration step may be conducted prior to simultaneous crystallization, wherein organic solvent or furanic compound is removed from the product mixture, e.g. by evaporation. Such a step will lower the organic/furanic ratio.

A crystallization step may be conducted prior to simultaneous crystallization, wherein either the organic solvent or the furanic compound is crystallized. This crystallization step (which may herein also be referred to as a regular crystallization step) is preferably conducted in a crystallizer other than the crystallizer in which simultaneous crystallization is conducted. Thus, the regular crystallization step makes it possible to use separate conditions for single crystallization and for simultaneous crystallization, such that for both crystallization types favorable conditions may be used. Furthermore, conducting a separate regular crystallization results in less crystals that need to be separated in the separation step conducted after simultaneous crystallization. The regular crystallization step is preferably a cooling crystallization, which is energetically favorable in view of the cooling already required for the simultaneous crystallization step. In case the organic solvent is crystallized, the organic/furanic ratio will decrease, whereas in case the furanic compound is crystallized, the organic/furanic ratio will increase. The regular crystallization step is preferably conducted just before simultaneous crystallization, but after water removal and filtration.

The eutectic points of many mixtures are known. However, if the eutectic point is not known, the skilled person will be able to make a rough estimate of the eutectic point based on the melting points of the organic solvent and the organic compound or by using theoretical equations like the van 't Hoff equation to simulate the phase diagram. Furthermore, the skilled person may determine the eutectic point by standard methods for experimental screening.

Just before simultaneous crystallization (*e.g.* the mixture fed to the crystallizer wherein simultaneous crystallization is conducted), the product mixture may have a concentration of dissolved furanic compound 0.001-100 wt%, preferably between 0.01-50 wt%, even more preferably between 1-30 wt%, even more between 10-30 wt%. The percentage is based on the total weight of the liquid in the crystallization mixture.

During simultaneous crystallization, the product mixture preferably comprises 10-50 wt.% crystals, more preferably 20-25 wt.% crystals. Such amount of crystals promotes the separation in the separation step. Furthermore, the mixture can still be easily handled at these crystal concentrations, *e.g.* by pumping, pouring or mixing. The crystal amounts particularly apply when simultaneous crystallization is conducted continuously.

The mother liquor that remains after removing the solvent crystals and furanic compound crystals from the slurry may be subjected to one or more further processing steps. The mother liquor typically comprises both organic solvent and the furanic compound. For example, at least part of the mother liquor may for example be fed back to simultaneous crystallization. Furthermore, at least part of the organic solvent may be reused in the conversion step.

In a preferred embodiment, the mother liquor is subjected to a (further) sugar recovery step. This is in particular desirable in case no steps have been taken earlier to remove sugar. In such a case, the mother liquor will contain a very high concentration of sugar since much of the solvent has been removed during simultaneous crystallization. As a result, sugar can be very efficiently removed from the mother liquor. For this purpose, nanofiltration is preferably used. In view of the difference in size between sugar and certain furanic compounds such as HMF and furfural, such a step may result in a retentate comprising the sugar dissolved in the organic solvent and a filtrate comprising the furanic compound dissolved in the organic solvent. Thus, sugar and furanic compound can be easily separated. At least part of the retentate may then be fed back to the first conversion step. At least part of the filtrate may then be fed back to the simultaneous crystallization step.

During simultaneous crystallization and/or regular crystallization, crystallization of the organic solvent or the furanic compound may be selectively promoted by crystallization seeds. Such seeds, which may be homogeneous or heterogeneous of nature, may be added to the product mixture at any suitable time.

After simultaneous crystallization, the furanic compound is recovered by separating the solid organic solvent and solid furanic compound. The inventors further found that the solid furanic compound and the solid organic solvent obtained by simultaneous crystallization can generally be easily separated, *e.g.* based on differences in density between the furanic compound and the organic solvent. As a result of the separation, both the compound and the solvent have a good purity. The organic solvent can be recycled (typically after melting) to the conversion reaction. Thus, the method of the invention has the advantage that the solvent and product can be efficiently recovered, without requiring complex and/or energy consuming downstream processing steps.

In case the solid organic solvent and solid furanic compound are separated based on their difference in density, separation may for example be conducted by gravity settling, by flotation, by centrifugation, by hydrocyclones or by any other known technique making use of the difference in density. After separation, the solid furanic compound crystals may be subjected to one or more purification steps. However, purification will require relatively less units of operation since the quality of the solid furanic compound crystals leaving the separation process will already be high and the amount of solvent and other components is relatively low.

In another aspect, the invention is directed to mixture comprising furanic compound crystals and organic solvent crystals. Such a mixture is obtainable by the simultaneous crystallization step described above. The mixture may further comprise the same furanic compound and organic solvent in liquid form. The mixture may comprise 10-50 wt.% crystals, preferably 20-25 wt.% crystals. The mixture may comprises at least 1 wt.%, preferably at least 3 wt.% of both the furanic compound crystals and the organic solvent crystals. The type of furanic compound and type of organic solvent that may be present in the mixture of the invention are the same as described above for the method of the invention.

## Claims

1. Method for preparing furanic compound crystals from sugars comprising the steps of
- converting sugar or sugar alcohol into a furanic compound in a reaction mixture comprising an organic solvent, thereby obtaining a product mixture comprising a furanic compound dissolved in said organic solvent and water;
- optionally further converting the furanic compound prior to crystallization; and
- simultaneously crystallizing the furanic compound and the organic solvent by cooling the product mixture, thereby obtaining a slurry comprising solvent crystals and furanic compound crystals; and
- separating the solvent crystals and the furanic compound crystals.

2. Method according to claim 1, wherein the sugar in the reaction mixture is dissolved in the organic solvent, which solvent is an aprotic solvent, wherein the aprotic solvent is preferably selected from the group consisting of trialkylphosphine oxides, dialkylsulfoxides and sulfolane, and which aprotic solvent is preferably dimethyl sulfoxide (DMSO).

3. Method according to claim 1, wherein the reaction mixture comprises two immiscible liquid phases, wherein the first liquid phase is an aqueous phase in which the sugar is dissolved and wherein the second liquid phase is an organic phase, into which the furanic compound is extracted, which two phases are separated to form the product mixture and an aqueous sugar mixture, wherein the organic solvent is preferably selected from the group of dialkyl sulfoxides, such as dibutyl sulfoxide; trialkyl phosphines, such as trioctylphosphine oxide and sulfolane.

4. Method according to any of the previous claims, wherein the organic solvent and the furanic compound are simultaneously crystallized by cooling the product mixture to a temperature equal or up to 10°C below the eutectic point of the mixture.

5. Method according to any of the previous claims, wherein the organic solvent and the furanic compound are simultaneously crystallized by cooling the product mixture to a temperature of -30 to 20°C

6. Method according to any of the previous claims, wherein the organic solvent and the furanic compound both have a melting point of at least -30°C, preferably at least 0°C, more preferably at least 15°C.

7. Method according to any of the previous claims, wherein the furanic compound crystals and organic solvent crystals are separated based on their difference in density.

8. Method according to any of the previous claims, wherein during simultaneous crystallization, the product mixture is in the form of a slurry and preferably comprises 10-50 wt.% crystals, more preferably 20-25 wt.% crystals.

9. Method according to any of the previous claims, further comprising shifting the ratio between the organic solvent and furanic compound in the product mixture prior to simultaneous crystallization by removing either organic solvent or furanic compound from the product mixture by conducting a concentration or cooling crystallization step prior to simultaneous crystallization.

10. Method according to any of the previous claims, wherein the reaction mixture further comprises a catalyst to promote the conversion of the sugar into the furanic compound and/or to promote the further conversion of the furanic compound if such a step is conducted.

11. Method according to any of the previous claims, wherein the furanic compound is selected from the group consisting of hydroxymethylfurfural (HMF); furfural (FF); furandicarboxylic acid (FDCA);
alkoxymethylfurfural, such as methoxymethylfurfural (MMF); and haloalkylfurfurals, such as 5-chloromethylfurfural; and wherein the furanic compound is preferably HMF.

12. Method according to any of the previous claims, wherein the furanic compound is further converted by modifying one or more side groups of the furan ring in the furanic compound, wherein the modification is selected from the group consisting of oxidation, reduction (such as hydrogenation or hydrogenolysis), removal (such as decarbonylation or decarboxylation), addition (such as carboxylation, esterification or etherification) and substitution (such as halogenation or amination); or wherein the furanic compound is further converted by subjecting the furanic compound to a cycloaddition reaction to obtain a mono-, di- or tri-substituted benzoic acid.

13. Method according to any of the previous claims, wherein the sugar is fructose or a modified fructose obtained by reacting fructose with a suitable alcohol and/or acid.

14. Mixture comprising furanic compound crystals and organic solvent crystals.

15. Mixture according to claim 14 obtainable by the simultaneous crystallization step of any of claims 1-13.
